# EUROPÄISCHE PATENTSCHRIFT

(11) **EP 0 789 549 B1**
(45) Veröffentlichungstag und Bekanntmachung des Hinweises auf die Patenterteilung: **10.01.2001**
(21) Anmeldenummer: 95937829.0
(22) Anmeldetag: 24.10.1995
(51) Int. Cl.: A61K 7/06

(54) **HAARBEHANDLUNGSMITTEL**
HAIR-TREATMENT AGENTS
PRODUITS DE SOINS CAPILLAIRES

(30) Priorität: 02.11.1994 DE 4438846
(43) Veröffentlichungstag der Anmeldung: 20.08.1997
(73) Patentinhaber: Henkel Kommanditgesellschaft auf Aktien, 40191 Düsseldorf (DE)
(72) Erfinder: EMMERLING, Winfried, D-41469 Neuss (DE); HOFMANN, Hans-Peter, D-40599 Düsseldorf (DE); SCHIEFERSTEIN, Ludwig, D-40882 Ratingen (DE)
(86) Internationale Anmeldenummer: EP9504160
(87) Internationale Veröffentlichungsnummer: WO9614049

(56) Entgegenhaltungen:
- DE-A- 4 225 045
- DE-A- 4 241 118

## Beschreibung

Die Erfindung betrifft Haarbehandlungsmittel in Form wäßriger Dispersionen von speziellen Polymeren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung bestimmter festigender Wirkstoffe aufbauen bzw. für einen längeren Zeitraum aufrechterhalten lassen.

Diese festigenden Wirkstoffe, bei denen es sich in der Regel um polymere Verbindungen handelt, können in übliche Haarreinigungs- oder -konditioniermittel eingearbeitet werden. In vielen Fällen ist es aber vorteilhaft, sie in Form spezieller Mittel wie Haarfestiger oder Haarsprays anzuwenden.

Es gibt nun in jüngster Zeit eine Reihe von Entwicklungen auf dem Haarkosmetikgebiet, die einen Bedarf an neuartigen festigenden Wirkstoffen bzw. neuen Formulierungsformen geweckt haben. Viele dieser Entwicklungen beruhen dabei nicht auf anwendungstechnischen Nachteilen oder Unzulänglichkeiten der bekannten Mittel, sondern z.B. auf Umweltschutz-Gesichtspunkten, gesetzlichen Auflagen oder anderen "nicht-technischen" Ursachen.

Es besteht dann die Aufgabe, entsprechende Mittel zu entwickeln, die hinsichtlich der anwendungstechnischen Eigenschaften, beispielsweise dem Sprühverhalten und der Trocknungszeit bei Haarsprays, die vom Verbraucher gesteckten Erwartungen erfüllen.

So wird insbesondere verstärkt ein Übergang von Mitteln auf Basis flüchtiger organischer Verbindungen, z.B. Alkoholen, zu Mitteln auf wäßriger Basis angestrebt. Dabei ist ein Hauptproblem die geringere Flüchtigkeit von Wasser im Vergleich zu den Alkoholen, was sich in längeren Trocknungszeiten auf dem Haar niederschlägt. Weiterhin ist aufgrund der häufig schlechteren Löslichkeit polymerer Verbindungen in wäßrigen Systemen diese Umstellung auch häufig mit dem Nachteil verbunden, daß beim Aufbringen der gewünschten Polymermenge auf das Haar Wasser zwangsläufig in solchen Mengen auf das Haar gelangt, daß die Trocknungszeiten unakzeptabel lang werden.

Dieser Nachteil tritt dagegen nicht auf, wenn diese nur unzureichend wasserlöslichen Polymeren gleichmäßig und stabil in dem Mittel dispergiert werden können. In diesem Fall gelangt bei der Anwendung mit der gewünschten Polymermenge nur eine weit niedrigere Wassermenge auf das Haar.

In den deutschen Patentanmeldungen P 44 14 423.7 und P 44 14 424.5 wurden daher Haarbehandlungsmittel in Form wäßriger Dispersionen vorgeschlagen, die als Polymere spezielle Polyacrylate enthalten.

Weiterhin ist u.a. aus der deutschen Offenlegungsschrift 42 25 045 bekannt, daß sich wasserlösliche und wasserdispergierbare Polyurethane bezüglicher einer Reihe von Effekten hervorragend zur Verwendung in Haarbehandlungsmitteln eignen. Dabei hat es sich aber als Nachteil erwiesen, daß gerade viele wasserdispergierbare, d.h. wasserunlösliche, Polyurethane nach der Anwendung nur sehr unzureichend wieder aus dem Haar auswaschbar sind.

Es wurde nun überraschenderweise gefunden, daß dieser Nachteil nicht auftritt, wenn das Haarbehandlungsmittel gleichzeitig bestimmte wasserlösliche Polymere enthält.

Gegenstand der Erfindung sind daher Haarbehandlungsmittel, enthaltend übliche kosmetische Bestandteile, in Form einer wäßrigen Dispersion eines wasserunlöslichen Polyurethans, die dadurch gekennzeichnet sind, daß die Mittel weiterhin ein wasserlösliches Polymer aus der Gruppe der nichtionischen, anionischen, amphoteren und zwitterionischen Polymeren enthalten.

Unter wäßrigen Dispersionen im Sinne der Erfindung sind solche Dispersionen zu verstehen, deren äußere Phase überwiegend aus Wasser besteht. Die äußere Phase kann darüber hinaus weitere, mit Wasser mischbare Lösungsmittel wie beispielsweise Ethanol und iso-Propanol enthalten; diese weiteren Lösungsmittel sind maximal in Mengen bis zu 20 Gew.-%, bezogen auf das gesamte Mittel, enthalten. Bevorzugt enthält die äußere Phase Wasser als einziges Lösungsmittel; eine weitere bevorzugte Ausführungsform enthält in der äußeren Phase, bezogen auf das gesamte Mittel, nicht mehr als 5 % weiterer Lösungsmittel.

Polyurethane sind wasserunlöslich im Sinne der Erfindung, wenn sie bei Raumtemperatur in Wasser zu weniger als 2,5 Gew.-% löslich sind.

Wasserlösliche Polymere im Sinne der Erfindung sind solche Polymeren, die bei Raumtemperatur in Wasser zu mehr als 2,5 Gew.-% löslich sind.

Die wasserunlöslichen Polymeren bestehen aus mindestens zwei verschiedenen Monomertypen,
- einer Verbindung (A) mit mindestens 2 aktiven Wasserstoffatomen pro Molekül und
- einem Di- oder Polyisocyanat (B).

Bei den Verbindungen (A) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (A) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und α,ω-Diamine auf Basis von langkettigen Alkanen oder Polyalkylenoxiden.

Polyurethane, bei denen die Verbindungen (A) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen. Polyesterole werden üblicherweise durch Modifizierung der Verbindung (A) mit Dicarbonsäuren wie Phthalsäure, Isophthalsäure und Adipinsäure erhalten.

Als Verbindungen (B) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fällen erfindungsgemäß besonders geeignet haben sich Polyurethane erwiesen, die wie folgt charakterisiert werden können:
- ausschließlich aliphatische Gruppen im Molekül
- keine freien Isocyanatgruppen im Molekül
- Polyether- und Polyesterpolyurethane
- anionische Gruppen im Molekül.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45%, auf und sind auch kommerziell erhältlich.

Die erfindungsgemäßen Haarbehandlungsmittel enthalten das unlösliche Polyurethan in Abhängigkeit vom Typ des Haarbehandlungsmittels, der keinen Einschränkungen unterliegt, bevorzugt in Mengen von 0,1 bis 30 Gew.-%, insbesondere 1 bis 20 Gew.-%, bezogen auf das gesamte Mittel.

Selbst bei höheren Konzentrationen, d.h. bei Konzentrationen oberhalb von etwa 10 %, zeichnen sich die erfindungsgemäßen Mittel im Vergleich zu Mitteln mit gelösten Polymeren durch sehr niedrige Viskositäten aus.

Weiterhin enthalten die erfindungsgemäßen Mittel ein wasserlösliches Polymer aus der Gruppe der nichtionischen, anionischen, amphoteren und zwitterionischen Polymeren.

Erfindungsgemäß bevorzugte wasserlösliche Polymere sind nichtionisch. Geeignete nichtionogene Polymere sind beispielsweise:
- Polyvinylpyrrolidone, wie sie beispielsweise unter der Bezeichnung Luviskol^{R} (BASF) vertrieben werden. Polyvinylpyrrolidone sind bevorzugte nichtionische Polymere im Rahmen der Erfindung.
- Vinylpyrrolidon/Vinylester-Copolymere, wie sie beispielsweise unter dem Warenzeichen Luviskol^{R} (BASF) vertrieben werden. Luviskol^{R} VA 64 und Luviskol^{R} VA 73, jeweils Vinylpyrrolidon/Vinylacetat-Copolymere, sind besonders bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{R} und Benecel^{R} (AQUALON) vertrieben werden.

Geeignete amphotere Polymere sind beispielsweise die unter den Bezeichnungen Amphomer^{R} und Amphomer^{R} LV-71 (DELFT NATIONAL) erhältlichen Octylacrylamid/Methylmethacrylat/tert.
Butylaminoethylmethacrylat/2-Hydroxypropylmethacrylat-Copolymere.
Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette^{R} (AMERCHOL) im Handel erhältlich sind.

Erfindungsgemäß geeignete anionische Polymere sind u. a.:
- Vinylacetat/Crotonsäure-Copolymere, wie sie beispielsweise unter den Bezeichnungen Resyn^{R} (NATIONAL STARCH), Luviset^{R} (BASF) und Gafset^{R} (GAF) im Handel sind.
- Vinylpyrrolidon/Vinylacrylat-Copolymere, erhältlich beispielsweise unter dem Warenzeichen Luviflex^{R} (BASF). Ein bevorzugtes Polymer ist das unter der Bezeichnung Luviflex^{R} VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold^{R} strong (BASF) vertrieben werden.

In den Fällen, in denen das wasserunlösliche Polyurethan ionische Gruppen enthält, hat es sich als zweckmäßig erwiesen, wenn das wasserlösliche Polymere nichtionogen oder von gleicher Ionogenität ist.

Die erfindungsgemäßen Haarbehandlungsmittel enthalten das wasserlösliche Polymer in Abhängigkeit vom Typ des Haarbehandlungsmittels bevorzugt in Mengen von 0,01 bis 20 Gew.-%, insbesondere 0,1 bis 10 Gew.-%, bezogen auf das gesamte Mittel.

Die wasserunlöslichen Polyurethane und die wasserlöslichen Polymere sind bevorzugt in einem Mengenverhältnis von 1:10 bis 10:1 in den erfindungsgemäßen Mitteln enthalten. Ein Mengenverhältnis von 2:1 bis 1:1 hat sich in vielen Fällen als besonders geeignet erwiesen.

Die weiteren Bestandteile der erfindungsgemäßen Haarbehandlungsmittel sind von der Art des Haarbehandlungsmittels abhängig. Prinzipiell umfassen die erfindungsgemäßen Formulierungen alle bekannten Arten von Haarbehandlungsmitteln wie z.B. Haarfestiger, Haarsprays, Fönwellen, Haarshampoos, Haarspülungen, Haarkonditioniermittel, Haarkuren, Dauerwellmittel, und Haarfärbemittel.

Bevorzugte erfindungsgemäße Haarbehandlungsmittel sind solche, die nach der Anwendung auf dem Haar verbleiben. Dies sind inbesondere Haarfestiger, Haarsprays und Fönwellen. Solche Mittel können mit Hilfe eines Treibmittels auch als Schaumaerosol formuliert sein.

Weitere Bestandteile der erfindungsgemäßen Mittel können beispielsweise sein:
- anionische Tenside, wie beispielsweise Fettalkylsulfate und -ethersulfate,
- kationische Tenside, wie beispielsweise quartäre Ammoniumverbindungen,
- zwitterionische Tenside, wie beispielsweise Betaine,
- ampholytische Tenside,
- nichtionogene Tenside, wie beispielsweise Alkylpolyglycoside und ethoxylierte Fettalkohole,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Gegenstand der Erfindung ist auch die Verwendung der erfindungsgemäßen Mittel zur Behandlung von Keratinfasern, insbesondere von Haaren.

Die folgenden Beispiele sollen die Erfindung näher erläutern.

### Beispiele

### 1. Anwendungstechnische Untersuchungen

### 1.1. Auswaschbarkeit

Haarsträhnen (# 6923 der Firma Fischbach und Miller, ca. 2 g, ca. 15 cm lang), die mit den zu untersuchenden Zubereitungen besprüht wurden (jeweils 1 g Spray) wurden nach 24 Stunden mit einer Tensidlösung (10 % Texapon^{R}N 28 (Natriumlaurylethersulfat, 28 % Aktivsubstanz in Wasser (HENKEL)) gewaschen, getrocknet und visuell auf Rückstände geprüft. Ergebnisse dieser Untersuchungen sind in Tabelle 1 zusammengestellt.

**Tabelle 1**

| (die Mengenangaben sind jeweils Gewichtsteile): | | |
|---|---|---|
| | Beispiel 1 | Vergleichsbeispiel 1 |
| Alberdingk^{R}U 500¹ | 10,0 | 10,0 |
| Luviskol^{R}VA 64² | 10.0 | - |
| Wasser | ad 100 | ad 100 |
| Auswaschbarkeit | gut | starke Rückstände |

| | | |
|---|---|---|
| ¹ anionische Polyether-Polyurethan-Dispersion (40% in Wasser) (ALBERDINGK BOLEY) | | |
| ² Vinylacetat-Vinylpyrrolidon-Copolymer (BASF) | | |

**Tabelle 2**

| (die Mengenangaben sind jeweils Gewichtsteile): | | |
|---|---|---|
| | Beispiel 2 | Vergleichsbeispiel 2 |
| Alberding^{R}U 500 | 5,0 | 5,0 |
| Amphomer^{R}LV 71³ | 5,0 | - |
| 2-Amino-2-methyl-propanol | q.s.⁴ | - |
| Wasser | ad 100 | ad 100 |

| | | |
|---|---|---|
| ³ Octylacrylamid-Acrylat-Copolymer (NATIONAL STARCH) | | |
| ⁴ zur 100%igen Neutralisation des Amphomer^{R} LV 71 | | |

### 1.2. Versprühbarkeit

Die Dispersionen gemäß Tabelle 1 waren gut versprühbar. Die Düsen verstopften auch nach längerem Gebrauch nicht.

### 2. Anwendungsrezepturen

### (alle Angaben sind Gewichtsteile)

### 2.1 Pumpspray

| | |
|---|---|
| Alberdingk U^{R}500 | 10,0 |
| Luviskol^{R}VA 64 | 10,0 |
| Panthenol | 0,1 |
| Parfümöl | 0,15 |
| Cremophor^{R}RH 40⁵ | 0,4 |
| Wasser | ad 100 |

| | |
|---|---|
| ⁵ ethoxyliertes, hydriertes Rizinusöl (CTFA-Bezeichnung: PEG-40-Hydrogenated Castor Oil) (BASF) | |

### 3.2 Pumpspray

| | |
|---|---|
| Alberdingk U^{R}500 | 5,0 |
| Amphomer^{R}LV 71 | 5,0 |
| AMP-95⁶ | 1,1 |
| Panthenol | 0,1 |
| Parfümöl | 0,15 |
| Cremophor^{R}RH 40 | 0,4 |
| Wasser | ad 100 |

| | |
|---|---|
| ⁶ 2-Amino-2-methyl-propanol (95 % in Wasser; CTFA-Bezeichnung: Aminomethyl Propanol) (ANGUS CHEMIE) | |

### 3.3 Pumpspray

| | |
|---|---|
| Alberdingk U^{R}500 | 5,0 |
| Luviskol^{R}K 90⁷ | 5,0 |
| Panthenol | 0,1 |
| Parfümöl | 0,15 |
| Cremophor^{R}RH 40 | 0,4 |
| Wasser | ad 100 |

| | |
|---|---|
| ⁷ Polyvinylpyrrolidon (95%-iges Pulver; CTFA-Bezeichnung: PVP) (BASF) | |

## Patentansprüche

1. Haarbehandlungsmittel, enthaltend übliche kosmetische Bestandteile, in Form einer wäßrigen Dispersion eines wasserunlöslichen Polyurethans, dadurch gekennzeichnet, daß das Mittel weiterhin ein wasserlösliches Polymer aus der Gruppe der nichtionischen, anionischen, amphoteren und zwitterionischen Polymeren enthält.

2. Haarbehandlungsmittel nach Anspruch 1, dadurch gekennzeichnet, daß das Polyurethan anionische Gruppen enthält.

3. Haarbehandlungsmittel nach Anspruch 1 oder 2, dadurch gekennzeichnet, daß das wasserlösliche Polymer nichtionisch ist.

4. Haarbehandlungsmittel nach Anspruch 3, dadurch gekennzeichnet, daß das wasserlösliche Polymere Polyvinylpyrrolidon oder ein Copolymeres, gebildet aus Vinylpyrrolidon- und Vinylester-Monomeren ist.

5. Haarbehandlungsmittel nach einem der Ansprüche 1 bis 4, dadurch gekennzeichnet, daß das Polyurethan in einer Menge von 0,1 bis 30 Gew.-%, insbesondere von 1 bis 20 Gew.-%, enthalten ist.

6. Haarbehandlungsmittel nach einem der Ansprüche 1 bis 5, dadurch gekennzeichnet, daß das wasserlösliche Polymere in einer Menge von 0,01 bis 20 Gew.-%, insbesondere von 0,1 bis 10 Gew.-%, enthalten ist.

7. Haarbehandlungsmittel nach einem der Ansprüche 1 bis 6, dadurch gekennzeichnet, daß weitere Lösungsmittel in Mengen bis max. 20 Gew.-%, bezogen auf das gesamte Mittel, enthalten sind.

8. Haarbehandlungsmittel nach einem der Ansprüche 1 bis 7, dadurch gekennzeichnet, daß es nach der Anwendung auf dem Haar verbleibt.

9. Verwendung eines Mittels nach einem der Ansprüche 1 bis 8 zur Behandlung von Haaren.

## Claims

1. A hair treatment composition in the form of an aqueous dispersion of a water-insoluble polyurethane containing typical cosmetic ingredients, characterized in that the composition additionally contains a water-soluble polymer from the group of nonionic, anionic, amphoteric and zwitterionic polymers.

2. A hair treatment composition as claimed in claim 1, characterized in that the polyurethane contains anionic groups.

3. A hair treatment composition as claimed in claim 1 or 2, characterized in that the water-soluble polymer is nonionic.

4. A hair treatment composition as claimed in claim 3, characterized in that the water-soluble polymer is polyvinyl pyrrolidone or a copolymer of vinyl pyrrolidone and vinyl ester monomers.

5. A hair treatment composition as claimed in any of claims 1 to 4, characterized in that the polyurethane is present in a quantity of 0.1 to 30% by weight and, more particularly, 1 to 20% by weight.

6. A hair treatment composition as claimed in any of claims 1 to 5, characterized in that the water-soluble polymer is present in a quantity of 0.01 to 20% by weight and, more particularly, 0.1 to 10% by weight.

7. A hair treatment composition as claimed in any of claims 1 to 6, characterized in that other solvents are present in quantities of up to at most 20% by weight, based on the composition as a whole.

8. A hair treatment composition as claimed in any of claims 1 to 7, characterized in that it is left on the hair after application.

9. The use of the composition claimed in any of claims 1 to 8 for treating hair.

## Revendications

1. Produit de soins capillaires qui contient des constituants cosmétiques usuels, sous la forme d'une dispersion aqueuse d'un polyuréthane insoluble dans l'eau,
caractérisé en ce que
le produit contient en outre un polymère soluble dans l'eau choisi dans le groupe des polymères non ioniques, anioniques, amphotères et zwiterioniques.

2. Produit de soins capillaires selon la revendication 1,
caractérisé en ce que
le polyuréthane contient des groupes anioniques.

3. Produit de soins capillaires selon la revendication 1 ou 2,
caractérisé en ce que
le polymère soluble dans l'eau est non ionique.

4. Produit de soins capillaires selon la revendication 3,
caractérisé en ce que
le polymère soluble dans l'eau est la polyvinylpyrrolidone ou un copolymère formé de monomères vinylpyrrolidone et d'ester vinylique.

5. Produit de soins capillaires selon l'une des revendications 1 à 4,
caractérisé en ce que
le polyuréthane est contenu en une quantité allant de 0,1 à 30 % en poids, en particulier de 1 à 20 % en poids.

6. Produit de soins capillaires selon l'une des revendications 1 à 5,
caractérisé en ce que
le polymère soluble dans l'eau est contenu en une quantité allant de 0,01 à 20 % en poids en particulier de 0,1 à 10 % en poids.

7. Produit de soins capillaires selon l'une des revendications 1 à 6,
caractérisé en ce que
des solvants supplémentaires sont contenus en des quantités allant jusqu'à au maximum 20 % en poids rapporté à l'agent total.

8. Produit de soins capillaires selon l'une des revendications 1 à 7,
caractérisé en ce que
celui-ci demeure après application sur les cheveux.

9. Utilisation d'un produit selon l'une des revendications 1 à 8, pour le traitement des cheveux.
